Europäisches Patentamt

European Patent Office

Office européen des brevets

(11) Veröffentlichungsnummer: **0 000 963**
**B1**

(12) **EUROPÄISCHE PATENTSCHRIFT**

(45) Veröffentlichungstag der Patentschrift: **23.06.82**

(21) Anmeldenummer: **78200106.9**

(22) Anmeldetag: **17.07.78**

(51) Int. Cl.³: **C 07 D 251/34,**
**C 08 G 18/80 //C09D3/72**

(54) **Isocyanuratgruppen und endständig-blockierte Isocyanatgruppen-enthaltende Verbindungen und Verfahren zu ihrer Herstellung.**

(30) Priorität: **20.07.77 DE 2732662**

(43) Veröffentlichungstag der Anmeldung:
**07.03.79 Patentblatt 79/5**

(45) Bekanntmachung des Hinweises auf die Patenterteilung:
**23.06.82 Patentblatt 82/25**

(84) Benannte Vertragsstaaten:
**BE DE FR GB LU NL**

(56) Entgegenhaltungen:
**DE - A - 2 502 934**
**DE - A - 2 644 684**
**FR - A - 2 292 724**
**FR - A - 2 299 354**
**NI - A - 7 403 748**
**US - A - 4 031 050**
**US - A - 4 055 551**

(73) Patentinhaber: **CHEMISCHE WERKE HÜLS AG**
**Postfach 1320**
**D-4370 Marl 1 (DE)**

(72) Erfinder: **Gras, Rainer, Dr.**
**An der Ziegelei 91**
**D-4690 Herne 2 (DE)**
Erfinder: **Wolf, Elmar, Dr.**
**Am Böckenbusch 3a**
**D-4690 Herne 2 (DE)**

(74) Vertreter: **Steil, Hanna, Dipl.-Chem. et al,**
**RSP PATENTE-PB 40 Holsterhauser Strasse 160**
**Postfach 2840**
**D-4690 Herne 2 (DE)**

Courier Press, Leamington Spa, England.

# Isocyanuratgruppen und endständig-blockierte Isocyanatgruppen-enthaltende Verbindungen und Verfahren zu ihrer Herstellung

Beschreibung und Beispiele

Die vorliegende Erfindung betrifft ein Verfahren zur Herstellung von neuen Verbindungen mit eingestelltem Isocyanuratgruppen-Gehalt, sowie die nach diesem Verfahren erhältlichen Verbindungen.

Die Herstellung von verkappten isocyanaten, auch Isocyanatabspalter genannt, ist bekannt und wird im Houben-Weyl, Methoden der organischen Chemie XIV/2 S. 61—70, beschrieben. Als Blockierungsmittel sind tertiäre Alkohole, Phenole, Acetessigester, Malonsäureester, Actylaceton, Phthalimid, Imidazol, Chlorwasserstoff, Cyanwasserstoff und ε-Caprolactam bekannt.

Diese verkappten Isocyanate besitzen die Eigenschaft, bei erhöhter Temperatur wie Isocyanate zu reagieren. Die Abspaltung erfolgt umso leichter, je saurer das H-Atom der Maskierungsgruppe ist. Derartige blockierte Isocyanate werden in der DT—A—21 66 423 beschrieben. Auch sind endständige blockierte Isocyanate, die zusätzlich noch Uretdiongruppen enthalten in der DE—A 25 02 934 beschrieben worden, es handelt sich um Dimerisate, nicht um Trimerisate, wobei nur letztere die anschließende Deblockierung katalysieren.

Aus FR—A 2 292 724 ist es bekannt, geblocktes Hexamethylendiisocyanat einzusetzen. Dieses ist aber hochviskos, also schlecht einbringbar.

Desweiteren ist es aus FR—A 2 299 354 bekannt, monomeres 3 - Isocyanatomethyl - 3,5,5 - trimethylcyclohexylisocyanat (Isophorondiisocyanat-IPDI) mit Dimethylketoxim zu blockieren, wobei aber dieses Produkt wegen seines neidrigen Schmelzpunktes nur bedingt einsatzfähig ist.

Schließlich bezieht sich NL—A 7 403 748 auf die Herstellung von Isocyanatrimeren, wobei aber IPDI als Diisocyanat nicht genannt ist.

Überraschenderweise finden sich in der Literatur keine Hinweise über Isocyanuratgruppen und endständig-blockierte Isocyanatgruppen aufweisendes Isophorondiisocyanat. Zwar sind blockierte aromatische Isocyanurate zur Herstellung hitzebeständiger abriebfester Urethan-Einbrennlacke, speziell für Elektrodrahtisolierungen, in JA—A 73.30453 aufgeführt. Die Herstellung so blockierter Isocyanuratgruppen enthaltender aliphatischer Polyisocyanate ist dagegen bis jetzt unbekannt.

Gegenstand der Erfindung ist ein Verfahren zur Herstellung von blockierten Isocyanatgruppen und Isocyanuratgruppen-enthaltenden Verbindungen aus Polyisocyanaten und monofunktionellen, aciden Wasserstoff-enthaltenden Blockierungsmitteln, dadurch gekennzeichnet, daß man zunächst 3 - Isocyanatomethyl - 3,5,5 - trimethyl-cyclohexylisocyanat in bekannter Weise in ein mehr als 2 freie Isocyanatgruppen enthaltendes Isocyanurat bestehendes Gemisch überführt und dieses Zwischenprodukt dann mit Dimethylketoxim als monofunktionelles, aciden Wasserstoff enthaltendes Blockierungsmittel bei 50—120°C umsetzt, wobei man das Dimethylketoxim in solchen Mengen einsetzt, daß auf ein NCO - Gruppen - Äquivalent ein Äquivalent Dimethylketoxim kommt. Eine Variante besteht darin, daß man das erhaltene Zwischenprodukt zunächst vom monomeren Polyisocyanat befreit und dann das praktisch monomerfreie Polyisocyanat anschließend in beschriebener Weise blockiert.

Gegenstand der Erfindung ist weiter ein, blockierte Isocyanatgruppen- und Isocyanuratgruppen-enthaltendes Gemisch, bestehend aus mindestens 10 Gew.-% eines mit Dimethylketoxim, vor der Blockierung mehr als 2 Isocyanatgruppen- enthaltendes Isocyanurats aus 3 - Isocyanatomethyl - 3,3,3 - trimethyl - cyclohexylisocyanat und gegebenenfalls einer auf 100 Gew.-% ergänzenden Menge des mit Dimethylketoxim blockierten, monomeren 3 - Isocyanatomethyl - 3,5,5 - trimethyl - cyclohexylisocyanats, wobei der Gesamtgehalt an unblockierten NCO-Gruppen bei <0,6 Gew.-% liegt.

Das monomer Polyisocyanat ist also mit dem identisch, welches zur Herstellung des Isocyanurats verwendet wurde. Das Isocyanurat kann auch ein Gemisch des trimeren und höheren Additionsproduktes des monomeren Polyisocyanats sein. Der Isocyanuratgruppen - Gehalt in den erfindungsgemäß hergestellten Verbindungen beträgt 2—8 Gew.-%.

Diese Ausgangsmaterialien für das erfindungsgemäße Verfahren werden dann in bekannter Weise der Trimerisierung unterworfen, wie beispielsweise in der GB—PS 1 391 066 und DT—A 23 25 826 beschrieben. Die Trimerisierung des cycloaliphatischen Isocyanates ist eine katalytische Reaktion. Als Katalysatoren können Metallverbindungen aus der Gruppe Salze und Basen und homöopolare Metallverbindungen wide Metallnaphthenate, Na-benzoat in DMF, Erdalkaliacetate, -formiate und -carbonate Metallalkoxide, $AlCl_3$ und Fe-Acetylacetonat eingesetzt werden. Besonders geeignet für die Trimerisierung ist das vorgeschlagene Katalysatorsystem aus N,N' - Endoäthylenpiperazin und Propylenoxid. Die Trimerisierung kann in Substanz oder in inerten organischen Lösungsmitteln vorgenommen werden. Zur Durchführung des Trimerisierungsverfahrens ist es wesentlich, die Reaktion bei einem bestimmten Isocyanatgehalt der Mischung abzubrechen, und zwar vorzugsweise dann, wenn 30—50% der NCO-Gruppen unter Trimerisierung reagiert haben. Das nicht umgesetzte Isocyanat wird dann durch Dünnschichtdestillation vom gebildeten Isocyanurat abgetrennt.

Bei dem erfindungsgemäßen Verfahren können die so zugängigen Isocyanuratisocyanate entweder als ausschließliche Isocyanatkomponente oder aber im Gemisch mit Isocyanurat-freiern Polyisocyanat eingesetzt werden. Der Zusatz von Isocyanuratgruppen - freien Polyisocyanat gestattet auf einfache Weise die Eigenschaften der Verfahrensprodukte, insbesondere ihren Schmelzpunkt, in gewünschter Weise zu variieren.

Es ist besonders vorteilhaft, beim erfindungsgemässen Verfahren als Isocyanuratkomponente das in situ hergestellte Triisocyanat-Gemisch einzusetzen, welches durch partielle Trimerisierung zugängig ist.

Bei den so erhaltenen Isocyanuratgruppen aufweisenden Polyisocyanat dern Triisocyanat, bzw. deoben Gemische mit Isocyanuratgruppen-freiem Polyisocyanat, handelt es sich um die oben als Zwischenprodukt bezeichnete Vorstufe zu den erfindungsgemäßen Verfahrensprodukten.

In einem weiteren Schritt des erfindungsgemäßen Verfahrens wird nun diese Zwischenstufe wie nachstehend beschrieben ohne weitere Modifizierung in das erfindungsgemässe Isocyanuratgruppen und endständig blockierte Isocyanatgruppen aufweisende Verfahrensprodukt überführt.

Als besonders geeignet hat sich das Dimethylketoxim erwiesen, das mit den Isocyanatgruppen enthaltenden Verbindungen bei Temperaturen über 50°C, vorzugsweise zwischen 80—120°C, leicht eine Additionsreaktion eingeht. Die so erhaltenen Additionsprodukte im Gemisch mit nichtflüchtigen primären Hydroxylgruppen aufweisenden Polyolen reagieren bei Temperaturen zwischen 100 und 200°C unter Freisetzung von Dimethylketoxim mit den nichtflüchtigen Polyolen unter Urethanbildung.

Zur Durchführung der Blockierungsreaktion wird im allgemeinen die Isocyanatkomponente vorgelegt und das Blockierungsmittel zugegeben. Die Reaktion kann in Substanz oder auch in Gegenwart geeigneter (inerter) Lösungsmittel durchgeführt werden. Die Blockierungsreaktion wird im allgemeinen bei 80—120°C durchgeführt. Das Dimethylketoxim wird in solchen Mengen eingesetzt, daß auf ein NCO-Gruppenäquivalent ein Äquivalent Blockierungsmittel kommt. Auch können die gebräuchlichen Isocyanat-Polyadditionsreaktion beschleunigenden Katalysatoren, wie Zinn-(II)-octoat mitverwendet werden. Die Katalysatoren werden in der Regel in einer Menge zwischen 0,001 und 1 Gew.-%, bezogen auf die Menge an Verbindungen mit gegenüber Isocyanaten reaktionsfähigen Wasserstoffatomen, eingesetzt.

Bei den erfindungsgemäßen Verfahrensduckten handelt es sich im allgemeinen um Verbindungen des Molekulargewichtsbereichs 300—3000, vorzugsweise 400—2000. Die Verfahrensprodukte besitzen Schmelzpunkte bzw. -bereiche von 30—200°C, vorzugsweise 60—170°C. Die bevorzugten erfindungsgemäßen Isocyanuratgruppen enthaltenden Polyisocyanate sind darüberhinaus durch einen Gehalt an Isocyanuratgruppen (berechnet als $(CO—N—)_3$) von 2 Gew.% bis 14 Gew.-%, vorzugsweise 3—10 Gew.-% und einem Gehalt an endständig in blockierter Form vorliegenden Isocyanatgruppen (berechnet als NCO) von 2—18, vorzugsweise 10—17 Gew.-% charakterisiert.

Die Verbindungen der vorstehend beschriebenen Erfindung eignen sich besonders wegen ihrer niedrigen Schmelzpunkte bei gleichzeitigen höheren Molekulargewichten als Kokatalysatoren für die anionische Polymerisation von ε-Caprolactam.

Die Verfahrensprodukte eignen sich weiter als Härter für Zerewittinoff-aktive Wasserstoffatome aufweisende höherfunktionelle thermoplastische Verbindungen. In Kombination mit derartigen Zerewitinoff-aktive Wasserstoffatome aufweisende Verbindungen bilden die Verfahrensprodukte oberhalb 120°C, vorzugsweise bei 160—200°C zu hochwertigen Kunststoffen aushärtbare Systeme. Das bedeutendste Anwendungsgebiet für derartige Systeme ist ihre Verwendung als Bindemittel für (PUR-)Pulverlacke. Auch sind die erfindungsgemäßen Verfahrensprodukte für die Herstellung von lagerstabilen Einkomponenten-Einbrennlacken (spez. für coil-coating) gut geeignet.

Die Erfindung wird durch die nachstehenden Beispiele illustriert:

Beispiel 1:
a. Herstellung des Triisocyanurats:
100 Gew.-T. Isophorondiisocyanat (IPDI) wurden mit 0,5 Gew.-T. des Katalysatorsystems aus 1,4 - -Diazobicyclooctan[2,2,2] (auch Dabco® gennant) und Propylenoxid 3 h bei 120°C erhitzt. Während dieser Zeit fiel der NCO-Gehalt von 37,8% (entsprechend 100% IPDI) auf 28,4% (50% IPDI-Umsatz). Zur Desaktivierung des Katalysators wurde das Reaktionsgemisch auf 40°C abgekühlt und bei dieser Temperatur 1/2 h mit Stickstoff gestrippt. Dabei veränderte sich der NCO-Gehalt des Reaktionsgemisches noch geringfügig auf 28,2%.

b. Blockierung des Triisocyanurats:
Zu 100 Gew.-T. dieses Isocyanato-isocyanurat-Gemisches wurden bei 100°C 49 Gew.-T. Dimethylketoxim portionsweise so zugegeben, daß die Reaktionstemperatur nicht über 120°C anstieg. Zur Vervollständigung der Reaktion wurde das Reaktionsgemisch noch 2 Stunden bei 120°C gehalten.

| | |
|---|---|
| Freies NCO (%) | <0,6 |
| Blockiertes NCO (%) | 18,92 |
| Smp. | 71—76 |
| Aufspalttemperatur | ca. 160 |
| Glasumwandlungstemperatur (DTA) | 41—62°C |

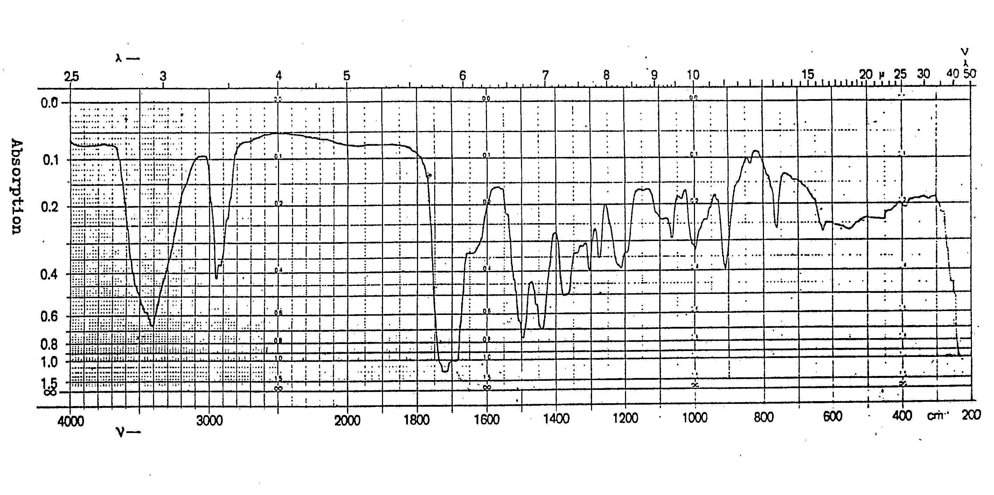

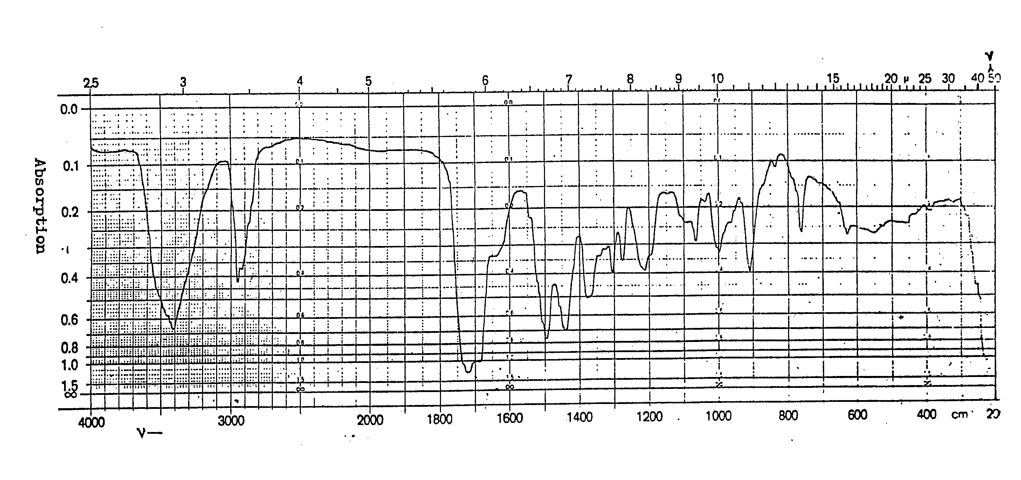

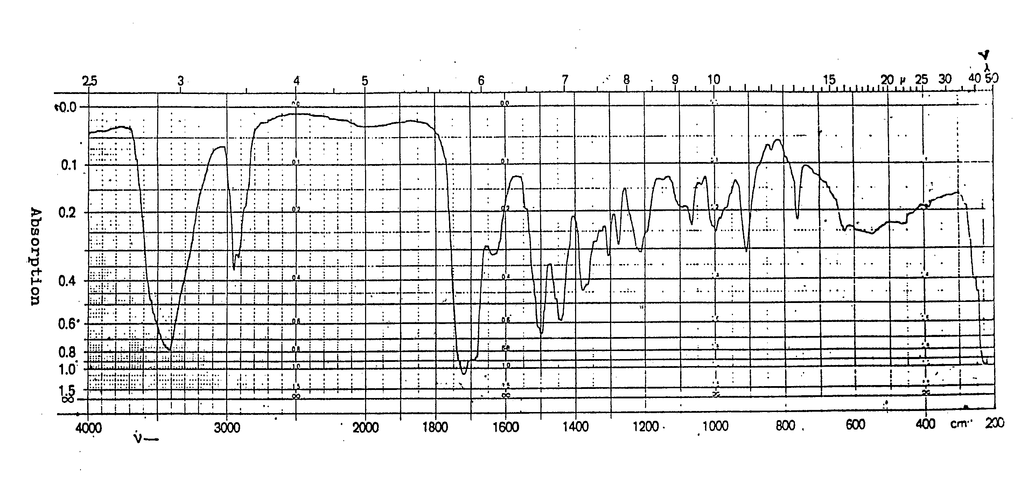

Das IR-Spektrum des Reaktionsproduktes zeigt die Abb. 1.

Beispiel 2:

a. Herstellung des Triisocyanurats:

100 Gew.-T. IPDI wurden mit 0,75 Gew.-T. eines Katalysatorsystems entsprechend Beispiel 1a 2 h bei 120°C erhitzt. In dieser Zeit fiel der NCO-Gehalt von 37,8% auf 29,4%. Zur Desaktivierung des Katalysators wurde bei 120°C und 30 Torr 15 Minuten evakuiert. Während dieser Zeit veränderte sich der NCO-Gehalt des Reaktionsgemisches auf 27%.

b. Blockierung des Triisocyanurats:

Zu 100 Gew.-T. dieses Isocyanatoisocyanurat-Gemisches wurden bei 100°C 46,9 Gew.-T. Dimethylketoxim portionsweise so zugegeben, daß die Reaktionstemperatur nicht über 120°C anstieg. Zur Vervollständigung der Reaktion wird das Reaktionsgemisch noch 2 Stunden bei 120°C gehalten.

| | |
|---|---|
| Freies NCO (%) | <0,5 |
| Blockiertes NCO (%) | 18,38 |
| Smp. | 75—82 |
| Aufspalttemperatur | ca. 160°C |
| Glasumwandlungstemperatur (DTA) | 50—69 |

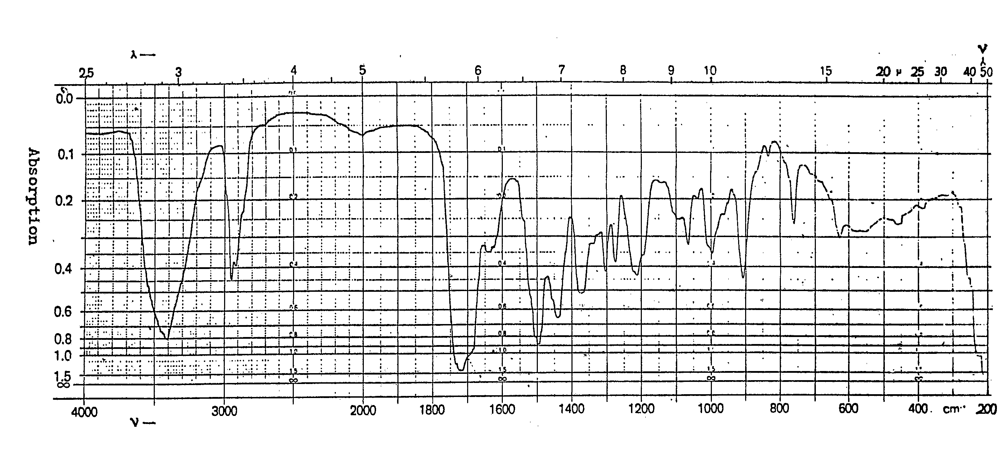

Das IR-Spektrum des Reaktionsproduktes zeigt die Abb. 2.

Beispiel 3:

a. Herstullung des Triisocyanurats:

100 Gew.-T. IPDI wurden mit 0,5 Gew.-T. des in Beispiel 1a beschriebenen Katalysators 4,5 h bei 120°C erhitzt. Das Fortschreiten der Trimerisierung wurde mit Hilfe des Brechungsindex, Viskosität oder NCO-Gehalt verfolgt. Bei einem NCO-Gehalt von 25,8% wurde eine halbe Stunde bei 20 Torr evakuiert. Nach Abkühlen hatte das Reaktionsgemisch einen NCO-Gehalt von 25%.

b. Blockierung des Triisocyanurates:

Zu 100 Gew.-T. dieses Isocyanatoisocyanurat-Gemisches wurden bei 120°C 43,4 Gew.-T. Dimethylketoxim langsam unter gutem Rühren zudosiert. Nach erfolgter Dimethylketoxinzugabe wurde das Reaktionsgemisch noch eine Stunde auf 130°C erhitzt.

| | |
|---|---|
| Freies NCO (%) | <0,6 |
| Blockiertes NCO (%) | 17,42 |
| Schmelztemperatur | 85—89°C |
| Aufspalttemperatur | ca. 160°C |
| Glasumwandlungstemperatur (DTA) | 51—62°C |

Das IR-Spektrum des Reaktionsproduktes zeigt die Abb. 3.

Beispiel 4:

a. Herstellung des Triisocyanurats:

Nach dem in 1a—3a beschriebenen Verfahren wurde aus IPDI ein Isocyanatoisocyanurat -Gemisch (mit desaktiviertem Kat.) hergestellt, dessen NCO-Gehalt 20,9% betrug.

b. Blockierung des Triisocyanurats:

Zu 100 Gew.-T. dieses Gemisches (NCO: 20,9%) wurden bei 140°C unter intensiver Rührung 36,3 Gew.-T. Dimethylketoxim so zugegeben, daß durch die Reaktionswärme das Gemisch bei 140°C gehalten wurde. Nach erfolgter Dimethylketoximzugabe wurde zur Vervollständigung der Umsetzung noch 1 h bei 140°C erhitzt.

| | |
|---|---|
| Freies NCO (%) | <0,6 |
| Blockiertes NCO (%) | 15,33 |
| Schmelztemperatur | 108—112 |
| Aufspalttemperatur | ca. 160 |
| Glasumwandlungstemperatur (DTA) | 79—91 |

Das IR-Spektrum des Reaktionsproduktes zeigt die Abb. 4.

**Patentansprüche**

1. Verfahren zur Herstellung von blockierten Isocyanatgruppen und Isocyanuratgruppenethaltenden Verbindungen aus Polyisocyanaten und monofunktionellen, aciden Wasserstoff-enthaltenden Blockierungsmitteln, dadurch gekennzeichnet, daß man zunächst 3 - Isocyanatomethyl - 3,5,5 - trimethyl - cyclohexylisocyanat in bekannter Weise in ein mehr als 2 freie Isocyanatgruppen enthaltendes Isocyanurat bestehendes Gemisch überführt und dieses Zwischenprodukt dann mit Dimethylketoxim als monofunktionelles, aciden Wasserstoff enthaltendes Blockierungsmittel bei 50—120°C umsetzt, wobei man das Dimethylketoxim in solchen Mengen einsetzt, daß auf ein NCO-Gruppen-Äquivalent ein Äquivalent Dimethylketoxim kommt.

2. Verfahren nach Anspruch 1, dadurch gekennzeichnet, daß man das erhaltene Zwischenprodukt von dem monomeren Polyisocyanat befreit und das dann praktisch monomerfreie Polyisocyanat anschließend in beschriebener Weise blockiert.

3. Blockierte Isocyanatgruppen- und Isocyanuratgruppenethaltendes Gemische bestehend aus

mindestens 10 Gew.-% eines mit Dimethylketoxim, vor der Blockierung mehr als 2 Isocyanatgruppen- ethaltendes Isocyanurats aus 3 - Isocyanatomethyl - 3,5,5 - trimethyl - cyclohexylisocyanat

und gegebenenfalls einer auf 100 Gew.-% ergänzenden Menge des mit Dimethylketoxim blockierten, monomeren 3 - Isocyanato - methyl - 3,5,5 - trimethyl - cyclohexylisocyanats,

wobei der Gesamtgehalt an unblockierten NCO-Gruppen bei <0,6 Gew.-% liegt.

## Claims

1. Process for production of blocked isocyanate groups and isocyanurate group-containing compounds of polyisocyanates and monofunctional, acid hydrogen-containing blocking agents, characterized in that one first converts 3 - isocyanatomethyl - 3,5,5 - trimethyl - cyclohexylisocyanate in known manner into a mixture consisting of an isocyanurate containing more than 2 free isocyanate groups and then transforms this intermediate product with dimethylketoxime as monofunctional, acid hydrogen-containing blocking agent at 50—120°C, where the dimethylketoxime is introduced in such quantities that to an NCO group-equivalent there corresponds an equivalent of dimethylketoxime.

2. Process according to Claim 1, characterized in that one frees the remaining intermediate product of monomeric polyisocyanate and blocks the then practically monomer-free polyisocyanate in the described manner.

3. Blocked isocyanate group- and isocyanurate group-containing mixture consisting of

at least 10 wt.% of an isocyanurate of 3 - isocyanatomethyl - 3,5,5 - trimethyl - cyclohexylisocyanate containing more than 2 isocyanurate groups before the blocking with dimethylketoxime

and, if necessary, a quantity making up the rest of the 100 wt.% of a dimethylketoxime-blocked monomeric 3 - isocyanatomethyl - 3,5,5 - trimethyl - cyclohexylisocyanate,

whereby the total content of unblocked NCO-groups is <0,6 wt.%.

## Revendications

1. Procédé de fabrication de composés contenant des groupes isocyanate bloqués et des groupes isocyanurate au départ de polyisocyanates et d'agents de blocage monofonctionnels contenant de l'hydrogène acide, caractérisé en ce qu'en premier lieu on convertit du 3 - isocyanatométhyl - 3,5,5 - triméthyl - cyclohexylisocyanate de manière connue en un mélange consistant en de l'isocyanurate contenant plus de 2 groupes isocyanate libres et en ce qu'on fait ensuite réagir ce produit intermédiaire avec de la diméthylcétoxime en tant qu'agent de blocage monofonctionnel contenant de l'hydrogène acide à 50—120°C, en utilisant la diméthylcétoxime en des quantités pour que par équivalent de groupe NCO corresponde un équivalent de diméthylcétoxime.

2. Procédé selon la revendication 1, caractérisé en ce qu'on débarrasse le produit intermédiaire obtenu du polyisocyanate monomère puis en ce qu'on bloque ensuite de la manière décrite le polyisocyanate alors pratiquement exempt de monomère.

3. Mélange contenant des groupes isocyanate bloqués et des groupes isocyanurate, consistant en au moins 10% en poids d'un isocyanurate contenant

plus de 2 groups isocyanate avant le blocage avec de la diméthylcétoxime, au départ de 3 - isocyanatométhyl - 3,5,5 - triméthylcyclohexyl - isocyanate,

et éventuellement en une quantité pour compléter à 100% en poids du 3 - isocyantométhyl - 3,5,5 - triméthylcyclohexyl - isocyanate monomère bloqué avec de la diméthylcétoxime,

la teneur totale en groupes NCO non bloqués étant inférieure à 0,6% en poids.

Absorption

2

0 000 963

0 000 963

3

**0 000 963**

Absorption

4